# EUROPÄISCHE PATENTANMELDUNG

(11) **EP 0 726 245 A1**
(43) Veröffentlichungstag der Anmeldung: **14.08.1996**
(21) Anmeldenummer: 96101171.5
(22) Anmeldetag: 29.01.1996
(51) Int. Cl.: C07C 67/317, C07C 69/74

(54) **Verfahren zur Herstellung von Fluorcyclopropan-Derivaten**

(30) Priorität: 10.02.1995 DE 19504317
(71) Anmelder: BAYER AG, 51368 Leverkusen (DE)
(72) Erfinder: Lui, Norbert, Dr., D-51061 Köln (DE)

(57) **Zusammenfassung**

Fluorcyclopropanderivate der Formel (I) 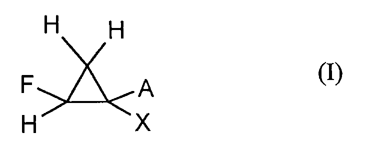 werden in besonders vorteilhafter Weise und mit einem hohen Anteil an cis-Isomeren (F und A stehen in cis-Stellung zueinander) erhalten, wenn man lineare Fluorpropanderivate der Formel (II) 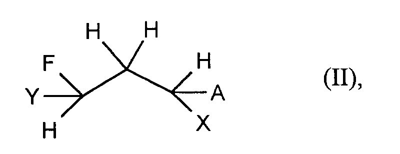 in wasserfreiem Milieu mit einer Base umsetzt. In den Formeln (I) und (II) bedeuten X und Y unabhängig voneinander Chlor, Brom oder Iod und A CN, CO₂R oder CONR'₂.

## Beschreibung

Die vorliegende Erfindung betrifft ein besonders vorteilhaftes Verfahren zur Herstellung von Fluorcyclopropan-Derivaten aus linearen Fluorpropan-Derivaten. Fluorcyclopropanderivate sind, insbesondere in ihrer cis-Form, wichtige Zwischenprodukte zur Herstellung von antibakteriell wirksamen Chinolonen.

Es ist bekannt, daß man 1-Fluor-2-chlor-2-carboxy-cyclopropan aus 2-Chlor-2-carboxy-1-cyclopropan-carbonsäure durch Fluorierung mit elementarem Fluor herstellen kann (siehe JP 05-301 827 A2). Bei diesem Verfahren werden cis- und trans-1-Fluor-2-chlor-2-carboxy-cyclopropan im Molverhältnis 3:4 erhalten. Nachteilig bei diesem Verfahren sind schlechte Ausbeuten, die geringe Selektivität der Bildung der cis-Verbindung, der erhebliche Aufwand, der für Arbeiten mit elementarem Fluor erforderlich ist und die Bildung von höher fluorierten Produkten, die praktisch nicht von den Monofluorprodukten abtrennbar sind.

Es wurde nun ein Verfahren zur Herstellung von Fluorcyclopropanderivaten der Formel (I) gefunden 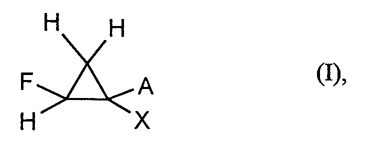 in der
- X: für Chlor, Brom oder Iod und
- A: für CN, CO₂R oder CONR'₂ stehen, wobei
- R: für eine Schutzgruppe und
- R': unabhängig voneinander jeweils für C₁-C₁₂-Alkyl, C₆-C₁₀-Aryl, C₂-C₁₂-Alkenyl oder C₇-C₁₂-Aralkyl, die gegebenenfalls substituiert sein können, stehen oder die NR'₂-Gruppe für einen gegebenenfalls substituierten Hydrazinrest steht,
das dadurch gekennzeichnet ist, daß man ein lineares Fluorpropanderivat der Formel (II) 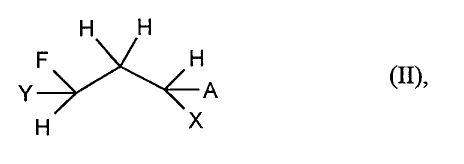 in der
- X und A: die bei Formel (I) angegebene Bedeutung haben und
- Y: unabhängig von X ebenfalls für Chlor, Brom oder Iod steht,
in wasserfreiem Milieu mit einer Base umsetzt.

In den Formeln (I) und (II) stehen X und Y unabhängig voneinander vorzugsweise für Chlor oder Brom und A vorzugsweise für CO₂R oder CONR'₂.

Als Schutzgruppen R kommen beispielsweise die in Frage, die in "Protective Groups in Organic Synthesis", 2nd ed., John Wiley and Sons Inc. 1991, S. 224 bis 270 als Schutzgruppen für Carboxylgruppen angegeben sind. Beispielsweise kann es sich bei R um C₁-C₁₂-Alkyl, C₂-C₁₂-Alkenyl, C₇-C₂₀-Alalkyl oder Triorganosilyl handeln. Alkyl, Alkenyl und Aralkyl können gegebenenfalls einfach oder mehrfach substituiert sein, beispielsweise mit Halogen, insbesondere Fluor, Chlor und/oder Brom, C₁-C₆-Alkoxy, Tetrahydropyranyl, Phenacyl, Nitrophenylsulfenyl und/oder Toluolsulfonyl. Als Aralkylreste seien insbesondere Benzyl, Diphenylmethyl und Triphenylmethyl genannt. Bei Triorganosilyl können die organischen Reste gleich oder verschieden und beispielsweise geradkettige und/oder verzweigte C₁-C₆-Alkylreste und/oder Phenylreste sein.

Soweit es sich bei R und R' um Alkyl- und Alkenylreste handelt können diese linear, verzweigt oder cyclisch sein. Beispiele sind Methyl, Ethyl, n-Propyl, i-Propyl, n-Butyl, i-Butyl, t-Butyl, Cyclopentyl, Cyclohexyl, Menthyl, Allyl und Cyclohexenyl.

Soweit R' für einen substituierten Rest und die NR'₂-Gruppe für einen substituierten Hydrazinrest stehen kommen als Substituenten beispielsweise diejenigen in Frage, die oben als Substituenten für Alkyl-, Alkenyl- und Aralkylgruppen angegeben sind. Hydrazinreste können 1, 2 oder 3 gleiche oder verschiedene Substituenten enthalten.

Soweit es sich bei R und R' um Aralkyl- und Arylreste handelt stehen diese vorzugsweise für Benzyl oder Phenyl.

Von den Triorganosilylresten sind Trimethylsilyl, Triethylsilyl und Phenyldimethylsilyl bevorzugt.

Wenn NR'₂ für einen Hydrazinrest steht sind ein unsubstituierter Hydrazinrest und mit 1 bis 3 gleichen oder verschiedenen Substituenten aus der Reihe Methyl, Ethyl, n-Propyl, i-Propyl und Phenyl substituierte Hydrazinreste bevorzugt.

Besonders bevorzugt stehen R und R' für C₁-C₆-Alkyl, einfach ungesättigtes C₂-C₆-Alkenyl oder Benzyl und R' zusätzlich für Phenyl.

Wenn A für CONR'₂ und NR'₂ nicht für einen Hydrazinrest stehen sind die beiden Reste R' vorzugsweise gleich.

Verbindungen der Formel (II) sind bekannt oder analog zu bekannten Verbindungen herstellbar. Beispielsweise kann man sie erhalten, indem man unter Kupferkatalyse Trichloressigsäureethylester an Vinylfluorid addiert, so 2,2-Dichlor-4-fluor-4-chlor-n-buttersäureester erhält und diesen zu 2-Chlor-4-fluor-4-chlor-n-buttersäureester hydriert (siehe Helv. Chim. Acta 63, 1947 bis 1957 (1980)).

Als Basen kommen z.B. starke Basen wie Hydride und Alkoholate von sekundären und tertiären Alkoholen in Frage, letztere vorzugsweise von C₃-C₆-Alkoholen, sowie Butyllithium (Buli) und Lithiumdiisopropylamid (LDA). Bevorzugt sind Alkalihydride und Alkalialkoholate von sekundären und tertiären C₃-C₆-Alkoholen, insbesondere Natriumhydrid, Lithiumhydrid, Natrium-t-butanolat und Kalium-t-butanolat. Starke Basen können bei allen Einsatzverbindungen der Formel (II) eingesetzt werden.

Wenn man Verbindungen der Formel (II) mit Y = Brom oder Iod einsetzt kann man auch schwächere Basen verwenden, beispielsweise Alkali- und Erdalkalicarbonate wie Natrium- und Kaliumcarbonat.

Vorzugsweise setzt man die Base mindestens in äquimolarer Menge, bezogen auf die Verbindung der Formel (II) ein, beispielsweise in Mengen von 1,05 bis 2 Äquivalenten, bezogen auf die Verbindung der Formel (II).

Das erfindungsgemäß erforderliche wasserfreie Milieu kann z.B. durch den Einsatz von inerten wasserfreien Lösungsmitteln realisiert werden. Als Lösungsmittel kommen z.B. Dialkylamide, N-alkylierte cyclische Amide, aromatische Kohlenwasserstoffe, sekundäre und tertiäre Alkohole und Ether in Frage. Einzelbeispiele sind Dimethylformamid, Dimethylacetamid, Benzol, Toluol, sekundäre und tertiäre C₃-C₆-Alkohole, Diethylether, N-Methylpyrrolidon, Methyl-t-butylether und Tetrahydrofuran. Von den Alkoholen ist t-Butanol besonders bevorzugt.

Bei der Auswahl der Lösungsmittel ist darauf zu achten, daß diese insbesondere gegenüber der verwendeten Base inert sind. Hydride, Buli und LDA kann man z.B. mit Dialkylamiden, N-Methylpyrrolidon, aromatischen Kohlenwasserstoffen und Ethern, nicht jedoch mit Alkoholen kombinieren. Alkoholate und schwächere Basen kann man prinzipiell mit allen genannten Lösungsmittel-Typen kombinieren. Vorzugsweise wendet man Alkoholate in Kombination mit Alkoholen an, insbesondere in Kombination mit dem Alkohol, der dem verwendeten Alkoholat entspricht.

Die Menge Lösungsmittel ist nicht kritisch. Bezogen auf 1 g Ausgangsprodukte (Verbindung der Formel (II) plus starke Base) kann man z.B. 0,5 bis 200 ml Lösungsmittel einsetzen.

Man kann die erfindungsgemäße Reaktion so durchführen, daß man das Edukt, gegebenenfalls zusammen mit Lösungsmittel, vorlegt und die starke Base, ebenfalls gegebenenfalls zusammen mit Lösungsmittel, zudosiert. Vorzugsweise legt man die starke Base und das Lösungsmittel vor und dosiert das Edukt zu.

Die Reaktionstemperaturen können beispielsweise im Bereich -20 bis +200°C liegen. Vorzugsweise liegen sie zwischen 0 und 150°C.

Die Reaktionszeit kann beispielsweise im Bereich 0,5 bis 20 Stunden liegen. Es ist im allgemeinen vorteilhaft, nach beendeter Zugabe des zweiten Reaktionspartners noch einige Zeit nachzurühren, z.B. 0,5 bis 20 Stunden.

Der Druck ist für das erfindungsgemäße Verfahren unkritisch. Man kann bei erhöhtem, normalem oder erniedrigtem Druck arbeiten, vorzugsweise arbeitet man bei Normaldruck.

Zur Aufarbeitung kann man z.B. das Reaktionsgemisch in Wasser oder auf Eis gießen, mit einem mit Wasser nicht mischbaren Lösungsmittel extrahieren und die organische Phase, gegebenenfalls nach Trocknung, destillativ aufarbeiten. Man kann das Reaktionsgemisch auch direkt destillativ aufarbeiten.

Erfindungsgemäß hergestellte Verbindungen der Formel (I) kann man z.B. gemäß der JP 06-157 518 A5 in 2-Fluorcyclopropancarbonsäureester überführen, die über die Stufe der entsprechenden Amine in antibakteriell wirksame Chinoloncarbonsäurederivate überführt werden können. Aus den cis-Verbindungen der Formel (I) werden dabei besonders wirksame Chinoloncarbonsäurederivate erhalten.

Das erfindungsgemäße Verfahren hat eine Reihe von Vorteilen: Mit ihm werden cis-Produkte in wesentlich höherer Selektivität erhalten als gemäß dem Stand der Technik. Die Selektivität der Bildung der cis-Produkte (Formel (I), F und A in cis-Stellung zueinander) beträgt z.B. über 80 %. Eine Bildung von höher fluorierten Nebenprodukten tritt beim erfindungsgemäßen Verfahren nicht auf, die Verfahrensdurchführung ist einfach, da kein elementares Fluor gehandhabt werden muß und die Ausbeuten sind gut.

Es ist ausgesprochen überraschend, daß diese Vorteile, insbesondere die hohe cis-Selektivität, mit dem erfindungsgemäßen Verfahren erzielbar sind. Wenn man völlig analog zu dem erfindungsgemäßen Verfahren verfährt, jedoch ein lineares Fluorpropanderivat einsetzt, das nur geringfügig von solchen der Formel (II) abweicht und statt X ein Wasserstoffatom enthält, so entstehen ausschließlich trans-Produkte (siehe Vergleichsbeispiel).

### Beispiele

Prozentangaben sind Gew.-%, soweit nichts anderes vermerkt ist.

### Beispiel 1 (zum Vergleich)

Zu einer Suspension von 0,6 g 60 %igem Natriumhydrid in Mineralöl in 30 ml Dimethylformamid wurden bei 10°C 2 g 4-Fluor-4-chlor-n-buttersäureethylester gelöst in 10 ml Dimethylformamid zugetropft. Nach 10-stündigem Rühren bei Raumtemperatur wurde die Reaktionsmischung auf Eis gegossen und mit Methylenchlorid mehrmals extrahiert. Die organischen Phasen wurden vereinigt, über Natriumsulfat getrocknet und das Methylenchlorid im Vakuum entfernt. Der Rückstand wurde ¹⁹F-NMR-spektroskopisch untersucht. Es hatte sich nur trans-2-Fluorcyclopropancarbonsäureethylester gebildet.

### Beispiel 2

Zu einer Suspension von 34 g 60 %igem Natriumhydrid in Mineralöl in 700 ml Dimethylformamid wurden bei 10°C 150 g 2,4-Dichlor-4-fluor-n-buttersäureethylester zugetropft. Nach 6-stündigem Rühren bei Raumtemperatur wurde die Reaktionsmischung auf Eis gegossen und mit Methylenchlorid mehrmals extrahiert. Die organischen Phasen wurden vereinigt, über Natriumsulfat getrocknet und das Lösungsmittel im Vakuum entfernt. Der Rückstand wurde bei 20 mbar destilliert. Es wurden 98 g 1-Chlor-2-fluorcyclopropancarbonsäureethylester erhalten. In dem Produkt betrug das cis-trans-Isomerenverhältnis (dieses bezieht sich auf die Stellung von Fluor zur Carboxylgruppe) 93:7.

### Beispiel 3

Zu einer Suspension von 4,1 g 60 %igem Natriumhydrid in Mineralöl in 50 ml N-Methylpyrrolidon wurden bei 20°C 15 g 2,4-Dichlor-4-fluor-butancarbonsäureethylester zugetropft. Nach 10-stündigem Rühren bei 50°C wurde die Reaktionsmischung auf Eis gegossen und mit Methylenchlorid mehrmals extrahiert. Die organischen Phasen wurden vereinigt, über Natriumsulfat getrocknet und das Lösungsmittel im Vakuum entfernt. Der Rückstand wurde bei 20 mbar destilliert. Es wurden 9,2 g 1-Chlor-2-fluorcyclopropancarbonsäure-ethylester isoliert. In dem Produkt betrug das cis-trans-Isomerenverhältnis (dieses bezieht sich auf die Stellung von Fluor zur Carboxylgruppe) 85:15.

## Patentansprüche

1. Verfahren zur Herstellung von Fluorcyclopropanderivaten der Formel (I) 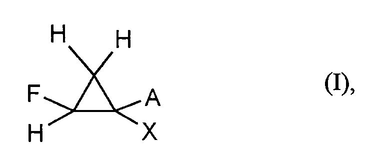 in der
X für Chlor, Brom oder Iod und
A für CN, CO₂R oder CONR'₂ stehen, wobei
R für eine Schutzgruppe und
R' unabhängig voneinander jeweils für C₁-C₁₂-Alkyl, C₆-C₁₀-Aryl, C₂-C₁₂-Alkenyl oder C₇-C₁₂-Aralkyl, die gegebenenfalls substituiert sein können, stehen oder die NR'₂-Gruppe für einen gegebenenfalls substituierten Hydrazinrest steht,
dadurch gekennzeichnet, daß man ein lineares Fluorpropanderivat der Formel (II) 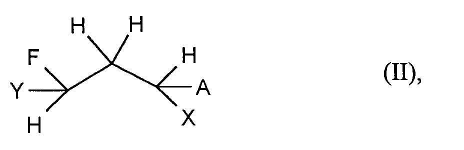 in der
X und A die bei Formel (I) angegebene Bedeutung haben und
Y unabhängig von X ebenfalls für Chlor, Brom oder Iod steht,
in wasserfreiem Milieu mit einer Base umsetzt.

2. Verfahren nach Anspruch 1, dadurch gekennzeichnet, daß in den Formeln (I) und (II) X und Y unabhängig voneinander für Chlor oder Brom und A für CO₂R oder CONR'₂ stehen.

3. Verfahren nach Ansprüchen 1 und 2, dadurch gekennzeichnet, daß R und R' für C₁-C₆-Alkyl, einfach ungesättigtes C₂-C₆-Alkenyl oder Benzyl und R' zusätzlich für Phenyl stehen.

4. Verfahren nach Ansprüchen 1 bis 3, dadurch gekennzeichnet, daß man als Basen Hydride, Alkoholate von sekundären oder tertiären Alkoholen, Butyllithium, Lithiumdiisopropylamid und/oder Alkali- und/oder Erdalkalicarbonate in mindestens äquimolarer Menge, bezogen auf die Verbindung der Formel (II) einsetzt.

5. Verfahren nach Ansprüchen 1 bis 4, dadurch gekennzeichnet, daß man als Basen Hydride, Butyllithium oder Lithiumdiisopropylamid und als Lösungsmittel Dialkylamide, N-alkylierte cyclische Amide, aromatische Kohlenwasserstoffe und/oder Ether einsetzt.

6. Verfahren nach Ansprüchen 1 bis 4, dadurch gekennzeichnet, daß man als Base ein Alkoholat eines sekundären oder tertiären Alkohols oder ein Alkali- oder Erdalkalicarbonat und als Lösungsmittel Dialkylamide, N-alkylierte cyclische Amide, aromatische Kohlenwasserstoffe, sekundäre oder tertiäre Alkohole und/oder Ether einsetzt.

7. Verfahren nach Ansprüchen 1 bis 6, dadurch gekennzeichnet, daß man es bei Temperaturen im Bereich -20 bis +200°C durchführt.
